# FASCICULE DE BREVET EUROPEEN

(11) **EP 2 059 188 B1**
(45) Date de publication et mention de la délivrance du brevet: **04.11.2015**
(21) Numéro de dépôt: 07823472.1
(22) Date de dépôt: 04.09.2007
(51) Int. Cl.: A61B 5/00, A61F 2/07, A61B 5/0215, A61B 5/07, A61F 2/90

(54) **ENDOPROTHÈSE ET PROCÉDÉ DE FABRICATION D'UNE ENDOPROTHÈSE.**
ENDOPROTHESE UND VERFAHREN ZUR HERSTELLUNG EINER ENDOPROTHESE
ENDOPROSTHESIS, AND METHOD FOR PRODUCING AN ENDOPROSTHESIS

(30) Priorité: 04.09.2006 FR 0607733
(43) Date de publication de la demande: 20.05.2009
(73) Titulaire: Université Pierre et Marie Curie (Paris 6), 75005 Paris (FR); Centre National de la Recherche Scientifique, 75016 Paris (FR); Assistance Publique - Hôpitaux de Paris, 75001 Paris (FR)
(72) Inventeur: LEPRINCE, Pascal, F-92500 Rueil Malmaison (FR); KAROUIA, Mourad, F-92200 Bagneux (FR); MAZEYRAT, Johan, F-92160 Antony (FR); ROMAIN, Olivier, F-91230 Montgeron (FR); LAGREE, Pierre-Yves, F-94250 Gentilly (FR); GARDA, Patrick, F-94320 Thiais (FR); KOKABI, Hamid, F-92390 Villeneuve la Garenne (FR)
(74) Mandataire: Blot, Philippe Robert Emile
(86) Numéro de dépôt international: PCT/FR2007/001431
(87) Numéro de publication internationale: WO 2008/029020

(56) Documents cités:
- EP-A- 0 897 690
- WO-A-02/098296
- WO-A-03/047419
- WO-A-2004/105637
- US-A1- 2003 229 388

## Description

La présente invention concerne un procédé de fabrication d'une endoprothèse tubulaire.

Une faiblesse dans une paroi d'une artère d'un être humain peut entraîner la dilatation locale de l'artère et former un anévrisme. L'anévrisme peut mettre en danger la vie du malade, à cause du risque de saignements (hémorragie) internes résultant d'une rupture de la paroi artérielle. Cette rupture est par exemple le résultat de l'application d'une pression sanguine sur la paroi fragilisée de l'artère.

Les anévrismes sont habituellement soignés, avant qu'une rupture ne se produise. Deux opérations chirurgicales peuvent être effectuées :
1. Une opération conventionnelle ou une ablation de l'anévrisme est pratiquée.
2. Une opération mini-invasive par voie endovasculaire via les artères fémorales. Cette opération consiste à exclure l'anévrisme de la circulation sanguine en plaçant une endoprothèse au niveau de l'anévrisme. L'endoprothèse est destinée à remplacer l'artère au niveau de l'anévrisme.

Bien que la procédure de pose de l'endoprothèse soit maintenant bien connue, il subsiste toujours un risque que des fuites se produisent, c'est-à-dire que l'exclusion de l'anévrisme ne soit pas parfaite, que ce soit juste après l'opération ou bien plusieurs mois après. Ces fuites sont appelées « endofuites de type I ou de type II suivant leur origine (migration de l'endoprothèse due à un défaut d'encrage, existence d'une circulation sanguine dans la poche anévrismale exclue due à des critères collatérales sus-rénales, etc).

Ainsi, il est primordial de surveiller l'endoprothèse et l'anévrisme afin de détecter les endofuites et, notamment, prévenir d'un risque de rupture post-opératoire. Il conviendrait dans ce cas de pratiquer l'opération la plus appropriée.

Une telle surveillance peut se faire par imagerie médicale, telle que l'imagerie à résonance magnétique mais elle est contraignante pour le malade.

Une autre possibilité, décrite dans US 6,159,156, est de placer dans l'anévrisme une sonde de pression identique, dont on vient recueillir la mesure grâce à un dispositif externe au patient. Ce document décrit également la fixation d'une sonde sur l'endoprothèse, afin que la sonde garde une position fixe dans l'anévrisme.

Ce document propose de déterminer un risque de rupture lorsque, quelque part dans l'anévrisme, une pression trop élevée ou ayant fortement changée par rapport à une précédente mesure est détectée.

Néanmoins, il a été remarqué que cette solution conduisait souvent à des conclusions erronées, notamment des fausses alertes. Cette méthode manque donc de précision.

Une autre possibilité est décrite dans US 2003229388

L'invention a pour but de pallier ce problème en améliorant la détection d'une fragilisation de la paroi anévrismal.

A cet effet l'invention a pour objet un procédé de fabrication d'une endoprothèse destinée à être implantée dans un anévrisme d'un patient résultant d'une déformation d'une paroi vasculaire, en particulier artérielle, comprenant des caractéristiques définies dans la revendication 1.

En effet, les inventeurs ont remarqué qu'il existe dans la poche anévrisme exclue la présence d'un caillot sanguin "thombus" qui va modifier la répartition de pression localement. Ainsi, l'existence à un endroit donné de l'anévrisme d'une pression d'une certaine valeur peut être tout à fait normale, bien qu'à un autre endroit de l'anévrisme elle soit élevée et risque d'entraîner une rupture. L'utilisation d'un seuil pour détecter un risque de rupture est donc inadaptée.

Grâce à l'invention, il est possible d'obtenir l'évolution de la mesure de pression réalisée pour chaque sonde, puisque les mesures transmises par chaque sonde sont différentiables. Ainsi, lorsque la pression mesurée par une même sonde augmente brutalement entre deux mesures consécutives, même lorsque les mesures des autres sondes n'ont pas varié, il existe une grande probabilité que la paroi anévrismale soit fragilisée et qu'une rupture soit imminente.

Une endoprothèse peut en outre comporter l'une ou plusieurs des caractéristiques suivantes :
- la caractéristique propre est comprise dans les données numériques envoyées dans le signal de transmission.
- le capteur est adapté pour fournir un signal électrique analogique, et l'endoprothèse comprend un amplificateur faible bruit pour amplifier ce signal analogique avant d'être introduit dans les moyens (pour transmettre la mesure de pression à l'appareil de surveillance,
- les moyens pour transmettre la mesure de pression sont destinés à générer un signal de transmission codant des données numériques, et l'endoprothèse comprend un convertisseur analogique/numérique pour convertir le signal analogique fourni par le capteur et amplifié,
- les sondes sont réparties en un ou plusieurs groupes de sondes, les sondes de chaque groupe étant fixées à intervalles réguliers autour de l'endoprothèse sur un même tronçon respectif de l'endoprothèse,
- le capteur de chaque sonde de la pluralité est un capteur directionnel, sa sensibilité étant maximale pour les pressions exercées perpendiculairement à sa surface de mesure,
- la déformation est une dilatation et les sondes sont orientées vers l'extérieur de l'enveloppe,
- la déformation est une rétraction et les sondes sont orientées vers l'intérieur de l'enveloppe,
- le signal de transmissions a une fréquence de porteuse comprise dans une des bandes fréquentielles libres définies dans la norme ISM.

Par ailleurs, l'invention a également pour objet un procédé de fabrication d'une endoprothèse destinée à être implantée dans un anévrisme d'un patient résultant d'une déformation d'une paroi vasculaire, en particulier artérielle, comprenant :
- une enveloppe tubulaire s'étendant suivant une direction, et
- une pluralité de sondes de pression fixées sur l'enveloppe, chaque sonde comprenant :
- un capteur pour réaliser une mesure de pression, et
- des moyens pour transmettre la mesure de pression à un appareil de surveillance placé à l'extérieur du patient,
les moyens pour transmettre la mesure de pression de chaque sonde étant adaptés pour générer un signal électromagnétique de transmission de la mesure de pression comportant au moins une caractéristique distinctive de la sonde dont ces moyens font partie,
le procédé étant caractérisé en ce qu'il comprend les étapes suivantes :
- fabriquer l'enveloppe,
- fixer les sondes de pression sur l'enveloppe.

Pour la fabrication d'une endoprothèse dont le capteur de chaque sonde de la pluralité est un capteur directionnel, sa sensibilité étant maximale pour les pressions exercées perpendiculairement à sa surface de mesure, le procédé étant caractérisé en outre par les étapes suivantes, un procédé selon l'invention peut en outre comporter une ou plusieurs des caractéristiques suivantes :
- obtenir une représentation visuelle de l'anévrisme du patient par imagerie médicale, fabriquer une enveloppe de forme adaptée à celle de l'anévrisme, localiser au moins un maximum local de pression exercée par des écoulements sanguins situés dans l'anévrisme sur la paroi de l'anévrisme du patient, et fixer au moins une des sondes de pression sur l'enveloppe en orientant la sonde vers l'extérieur de l'enveloppe, de telle sorte que la sonde soit orientée vers le maximum local de pression lorsque l'endoprothèse est implantée dans l'anévrisme,
- déduire une modélisation de l'anévrisme à partir de la représentation visuelle, et localiser par calcul le ou les maximum locaux de pression exercée sur la paroi de l'anévrisme modélisé.,
- la représentation visuelle est une vue de scanner ou d'IRM,
- pour fixer la sonde de pression sur l'endoprothèse, le procédé comprend les étapes suivantes : choisir sur la modélisation de l'anévrisme une configuration de pose de l'endoprothèse, telle que l'endoprothèse s'étende le long d'une direction d'écoulement du flux sanguin, déterminer une zone de fixation de capteurs sur l'enveloppe de l'endoprothèse telle que le maximum local se trouve à la perpendiculaire de la zone de fixation par rapport à l'enveloppe, et fixer la sonde de pression sur la zone de fixation en orientant la sonde perpendiculairement à l'enveloppe vers l'extérieur de l'endoprothèse.
- déterminer une tranche à risque de l'anévrisme, perpendiculaire à la direction d'écoulement et formant le maximum local, et déterminer, le long de la direction de l'enveloppe dans la configuration de pose de l'endoprothèse, un tronçon de l'enveloppe délimité par la tranche à risque, le tronçon formant la zone de fixation de la sonde de pression,
- la tranche à risque présente une épaisseur correspondant à une dimension que présente la sonde de pression le long de la direction de l'enveloppe lorsque la sonde est fixée,
- fixer plusieurs sondes sur le tronçon de fixation autour de la direction d'endoprothèse,
- les sondes sont réparties autour de la direction de l'enveloppe à intervalles réguliers.

L'invention sera mieux comprise à la lecture de la description qui va suivre, donnée uniquement à titre d'exemple et faite en se référant aux dessins annexés, parmi lesquels :
- la figure 1 est une vue en coupe d'un anévrisme ;
- la figure 2 est une vue de face d'une endoprothèse munie de sondes de pression ;
- la figure 3 est un diagramme représentant les éléments d'une sonde fixée sur l'endoprothèse ;
- la figure 4 est une vue de coupe d'une modélisation de l'anévrisme de la figure 1 ;
- la figure 5 est une vue similaire à celle de la figure 4, avec l'endoprothèse modélisée ;
- la figure 6 est une vue de dessus de l'endoprothèse de la figure 2 ;
- la figure 7 est une vue similaire à celle de la figure 1, avec l'endoprothèse posée dans l'anévrisme ;
- la figure 8 est un schéma-blocs d'un procédé de fabrication de l'endoprothèse selon l'invention; et
- la figure 9 est un schéma-blocs d'un procédé d'utilisation de l'endoprothèse implantée.

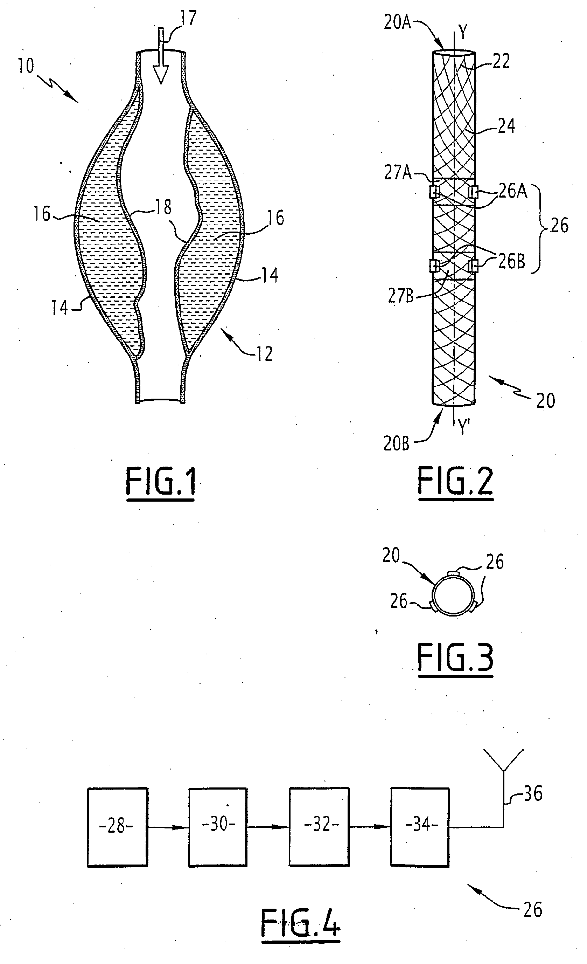

En référence à la figure 1, l'artère aortienne 10 d'un patient présente un anévrisme 12 résultant d'une dilatation d'une partie d'une paroi 14 formant l'artère 10.

L'anévrisme 12 tel que représenté comprend un caillot sanguin mou, ou thrombus 16, s'étendant depuis la paroi artérielle 14 vers le centre de l'anévrisme 12. La partie du thrombus 16 en contact avec du sang en circulation (représenté par la flèche 17) forme souvent une pellicule, ou intemna 18.

L'endoprothèse 20 représentée sur la figure 2 est destinée à exclure l'anévrisme 12 du sang en circulation 17. L'endoprothèse 20 comprend un treillis tubulaire 22 noyé dans un tissu ou film 24 extensible étanche au sang et biocompatible tel qu'un élastomère. L'ensemble du treillis 22 et du film 24 forme donc une enveloppe tubulaire 22, 24 de l'endoprothèse 20. Le treillis 22 est constitué d'acier inoxydable ou d'un alliage ayant des propriétés de ressort, de sorte que l'endoprothèse 20 est auto-extensible. Une telle endoprothèse est couramment désignée par le terme anglais « stent ».

L'endoprothèse 20 est susceptible de se déformer spontanément d'un état comprimé, dans le cas présent d'un petit diamètre, à un état dilaté, dans lequel elle présente un diamètre supérieur, cet état dilaté constituant son état de repos. Du fait de son élasticité, l'endoprothèse 20 est destinée, une fois posée, à s'appliquer contre la surface interne de l'artère 10 de chaque côté de l'anévrisme 12, constituant ainsi une gaine intérieure.

L'endoprothèse 20 s'étend, avant d'être posée, le long d'une direction Y-Y'. Dans l'exemple illustré, l'endoprothèse 20 est « simple », c'est-à-dire qu'elle ne forme qu'un seul conduit rectiligne. Dans d'autres modes de réalisation, suivant la localisation de l'anévrisme 12 le long de l'artère 10, l'endoprothèse peut être « double ou bifurquée », c'est-à-dire qu'elle délimite d'un côté un seul conduit se divisant pour donner naissance, de l'autre côté, à deux conduits. Dans ce cas, il est évident que l'endoprothèse 20 s'étendra le long de plusieurs directions différentes, suivant le conduit considéré.

L'endoprothèse 20 comprend en outre une pluralité de sondes 26 fixées sur l'enveloppe 20, 24 et orientées vers l'extérieur de celle-ci.

Les sondes 26 sont réparties en deux groupes, respectivement 26A et 26B. Les sondes de chaque groupe 26A, 26B sont fixées sur un même tronçon, respectivement 27A et 27B de l'endoprothèse 20, autour de sa direction Y-Y' sur sa périphérie extérieure. Chaque groupe de sondes a une position définie le long de l'endoprothèse 20. Leur position est par exemple repérée par rapport à l'une des extrémités 20A, 20B de l'endoprothèse 20, telle que l'extrémité supérieure 20A par laquelle le sang en circulation 17 est destiné à entrer.

Comme cela est visible sur la figure 3, les sondes 26 de chaque groupe sont fixées autour de l'endoprothèse 20 à intervalles égaux.

En référence à la figure 4, chaque sonde de pression 26 comporte un capteur de pression 28 formant un transducteur pour délivrer une tension analogique fonction de la pression mesurée. Le capteur 28 mesure la pression de manière locale, au voisinage d'une surface de mesure (non représentée). Néanmoins, le capteur 28 est capable de mesurer la présence d'endofuites située à distance de la surface de mesure, car la pression générée par les endofuites entraîne une surpression au niveau de la surface de mesure.

Le capteur 28 est directionnel, sa sensibilité étant maximale pour les pressions exercées perpendiculairement à sa surface de mesure.

Comme cela sera expliqué par la suite, chaque sonde 26 est fixée à l'endoprothèse 20 de manière que le capteur 28 soit orienté perpendiculairement à l'enveloppe 22, 24, c'est-à-dire de manière que la surface de mesure soit parallèle à l'enveloppe 22, 24.

La tension délivrée par le capteur 28 est de préférence de type différentiel, de manière à limiter le bruit de mesure.

Le capteur 28 mesure une valeur « absolue » de pression, c'est-à-dire par rapport à une référence fixe (pression atmosphérique) prédéterminée. Les sondes 26 se distinguent en cela des sondes existantes mesurant des pressions relatives, prises en deux points différents à l'intérieur de l'anévrisme. De préférence, la gamme de mesure du capteur 28 s'étend de 10 à 300 mm de mercure.

La tension délivrée par le capteur 28 est introduite dans un amplificateur faible bruit 30 afin de mettre en forme le signal analogique. L'amplificateur faible bruit 30 comporte un amplificateur d'instrumentation (non représenté) possédant un taux de réjection en mode commun supérieur de préférence à 80, et un filtre passe bas (également non représenté) dont la fréquence de coupure est choisie de façon à pouvoir récupérer la fréquence fondamentale du signal mais aussi suffisamment d'harmoniques pour récupérer la pression systolique. En effet, il a été remarqué qu'un taux de réjection inférieur entraînait la présence de bruit.

La fréquence de coupure est déterminée en considérant que la fréquence d'un patient a une valeur entre 0,3 et 2 Hz et en choisissant un facteur de sécurité de 5.

Chaque sonde 26 comprend ensuite un convertisseur analogique/numérique 32 pour la conversion du signal analogique mis en forme par l'amplificateur faible bruit 30 en données numériques. De préférence, le convertisseur analogique/numérique 32 possède une résolution supérieure à 16 bits et une fréquence d'échantillonnage supérieure à 100 Hertz. En effet, il est nécessaire, d'après le théorème de Shannon, de prendre une fréquence d'échantillonnage qui soit au moins deux fois supérieure à 2 Hz (fréquence physiologique caractéristique). Ici, on prend un facteur de sécurité de 50, soit 100 Hz. L'architecture du convertisseur 32 est du type à double rampes, ou de préférence, car plus rapide, à approximation successive.

Les données numérisées sont introduites dans un transpondeur 34 destiné à émettre un signal électromagnétique par l'intermédiaire d'une antenne 36 à destination de l'extérieur du corps du patient.

L'architecture électronique de chaque sonde comprend tous les éléments (transducteur, convertisseur analogique numérique et transpondeur) qui font qu'elle peut être vue comme une sonde de pression de type RFID (Radio Frequency Identification).

L'antenne 36 possède une fréquence porteuse comprise dans une des bandes fréquentielles libres définies dans la norme ISM, de l'anglais "Industrial Scientific and Médical" pour l'émission de données, qui est spécifique à chaque sonde 26. Ainsi, les signaux émis par chacune des sondes 26 sont différenciables les uns des autres au moyen des fréquences porteuses utilisées. Il est ainsi très facile de sélectionner les signaux émis par un capteur particulier. En variante, les signaux sont différenciables de par les données numériques envoyées. Cela permet de réduire le coût de fabrication, car seul un paramètre logiciel est à changer entre les sondes, c'est-à-dire dans les bandes ISM (par exemple à 125 kHz, 13,56 MHz, 480 MHz, 900 MHz ou 2,45 GHz), tandis que dans la variante précédente, la structure elle-même des sondes devait être modifiée. Les deux variantes peuvent être combinées.

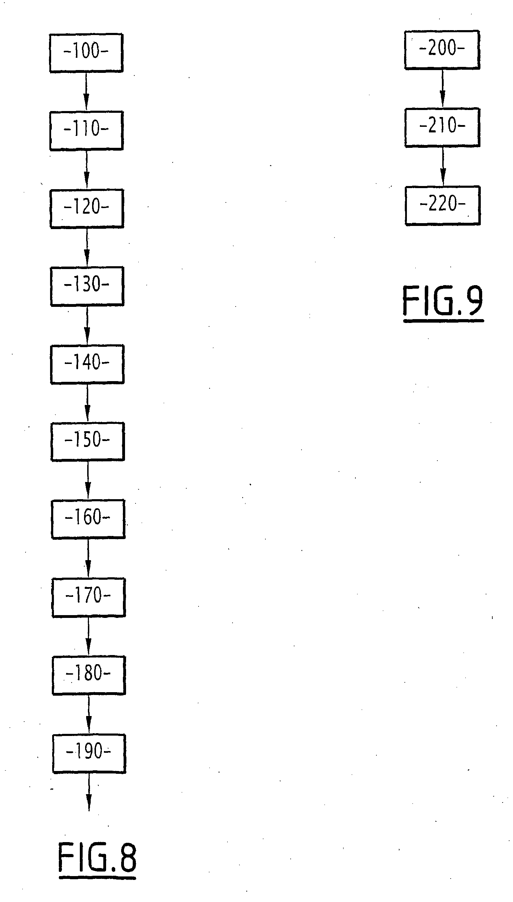

En référence à la figure 8, on va décrire à présent un procédé de fabrication de l'endoprothèse 20, et notamment comment sont positionnés les groupes de sondes 26A et 26B le long de l'endoprothèse 20.

Au cours d'une première étape 100, une représentation visuelle de l'anévrisme 12 du patient à traiter est obtenue par imagerie médicale. Cette représentation est, de préférence, une vue de scanner ou d'IRM, donnant une image de l'anévrisme similaire, par exemple, à celle de la figure 1.

Le choix entre IRM et scanner se fait notamment en fonction des matériaux constituant l'endoprothèse.

Il s'en suit une étape 110 de fabrication de l'enveloppe 22, 24 de forme adaptée à celle de l'anévrisme 12.

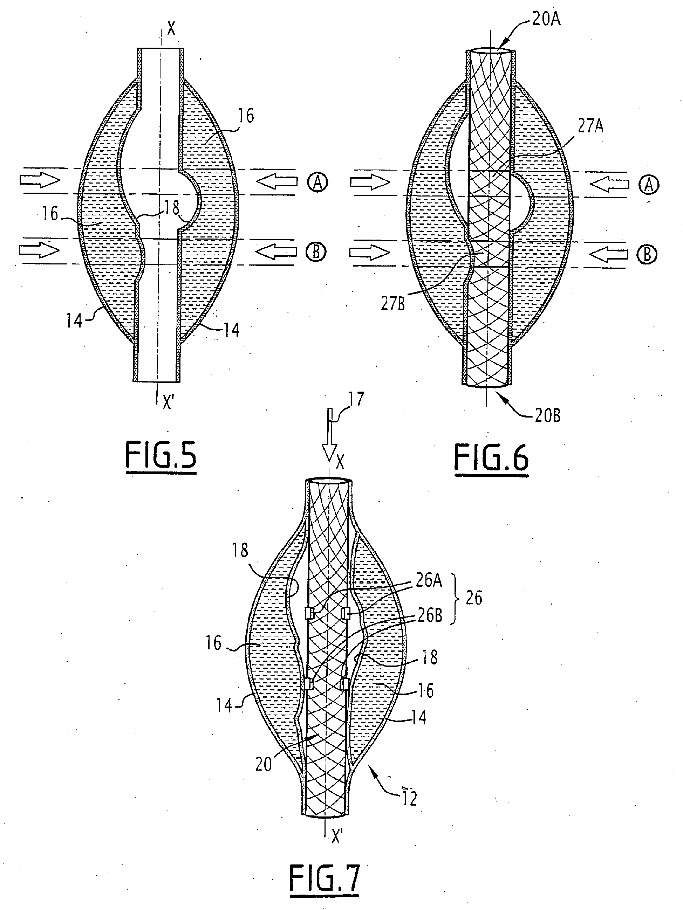

Au cours d'une étape 120, une modélisation de l'anévrisme est déduite à partir de la représentation visuelle. Une modélisation possible est par exemple représentée sur la figure 5. Des formes simples sont utilisées pour correspondre à la forme générale de l'anévrisme réel. Par ailleurs, on détermine également au cours de cette étape 120 une direction générale d'écoulement X-X' du flux sanguin 17. Cette direction d'écoulement X-X' correspond de préférence à l'écoulement que le sang suivrait s'il n'y avait pas d'anévrisme. Dans l'exemple décrit, cette direction est rectiligne, mais elle pourrait être courbée, voire se diviser dans le cas d'un anévrisme au niveau d'un embranchement.

La modélisation est de préférence réalisée en élasticité non linéaire. Au cours de la modélisation, les différents tissus sont représentés par une suite de milieux élastiques de coefficients mécaniques différents. Par exemple, on choisira un coefficient élastique différent pour la paroi artérielle de l'anévrisme 14, pour le thrombus 16, pour l'intemna 18 et pour le sang 17 en circulation dans l'artère 10.

Le choix des coefficients mécaniques différents est obtenu, dans un mode de réalisation, à partir du traitement de patients précédents, comme cela sera expliqué par la suite.

Le procédé de fabrication continue par une étape 130 de réalisation d'une échographie, puis par une étape 140 de mesure du flux sanguin 17 à l'entrée de l'anévrisme 12, à partir de l'échographie.

Grâce à la modélisation des tissus et à la mesure du flux sanguin, on établit et on résout les équations de mécanique de fluide concernant l'écoulement sanguin dans l'anévrisme au cours d'une étape 150.

Cette résolution permet d'obtenir, au cours d'une étape 160, les maximums locaux de pression exercés sur la paroi artérielle 14 de l'anévrisme 12. Il s'agit des endroits les plus fragiles, où le risque de rupture est le plus important.

Dans le mode de réalisation décrit, l'étape 160 de localisation des maximums locaux consiste à déterminer la position le long de la direction d'écoulement X-X' d'une tranche à risque de l'anévrisme perpendiculaire à cette direction X-X'. En effet, les inventeurs ont remarqué que les zones fragilisées de la paroi artérielle 14 dépendaient de la géométrie spatiale de celle-ci et étaient généralement concentrées en anneaux autour de la direction d'écoulement X-X'. Ceci est du au fait, notamment, que les endofuites génèrent une surpression non seulement à l'endroit où elles apparaissent, mais également, dans une moindre mesure, tout autour de la direction d'écoulement X-X'.

On obtient ainsi plusieurs tranches à risque, chacune correspondant à une zone fragilisée de l'anévrisme 12. Dans l'exemple représenté, deux tranches ont été déterminées, référencées A et B. De préférence, la hauteur de ces tranches correspond sensiblement à la dimension que présente chaque sonde 26 le long de l'endoprothèse, lorsque la sonde est fixée.

En référence à la figure 6, au cours d'une étape 170 on fait correspondre la direction Y-Y' de l'endoprothèse 20 avec la direction d'écoulement X-X', puis on choisit sur la modélisation de l'anévrisme 12 une configuration de pose de l'endoprothèse, c'est-à-dire notamment sa position le long de la direction d'écoulement X-X'.

On en déduit, au cours d'une étape 180, la position des tronçons de fixation 27A, 27B des groupes de sondes 26A, 26B, ces tronçons de fixation correspondant à l'intersection des tranches à risque A, B avec l'enveloppe 22, 24 de l'endoprothèse 20.

Chaque tronçon 27A, 27B est localisé le long de l'endoprothèse par sa distance, le long de la direction Y-Y' de l'endoprothèse 20, par rapport à l'une des extrémités de l'endoprothèse 20, par exemple l'extrémité supérieure 20A.

Le procédé comprend alors une étape 190 de fixation d'une pluralité de sondes sur chaque tronçon 17A, 27B, chaque pluralité de sondes fixées sur un même tronçon formant un groupe de sondes 26A, 26B. Les sondes 26A, 26B d'un même groupe sont fixées autour de la direction de l'endoprothèse 20, à intervalles réguliers. Chaque sonde 26 est orientée perpendiculairement à l'enveloppe de l'endoprothèse 20, c'est-à-dire perpendiculairement à la direction Y-Y'. Ainsi, la pression peut être mesurée dans toutes les directions transversales à la direction Y-Y', dans la tranche à risque considérée de l'anévrisme.

De préférence, les sondes sont fixées par collage sur l'enveloppe de l'endoprothèse. La colle utilisée sera de type biologique. Ce type de fixation permet de ne pas fragiliser le tissu biocompatible 24, comme c'est par exemple le cas lorsque la sonde est cousue sur l'enveloppe.

L'endoprothèse 20 munie des sondes 26A et 26B, est implantée par des méthodes habituelles afin de la disposer dans sa configuration de pose choisie sur la modélisation. L'endoprothèse posée est représentée sur la figure 7.

On remarquera que, grâce à l'invention, l'efficacité de l'endoprothèse une fois posée n'est pas sensible à l'orientation angulaire de l'endoprothèse autour de la direction d'écoulement X-X', puisque les sondes 26 sont réparties de manière égale autour de cette direction. Cela facilite grandement la pose de l'endoprothèse qui peut posséder une orientation angulaire quelconque.

On va à présent décrire un procédé d'utilisation de l'endoprothèse posée 20.

Suite à la pose de l'endoprothèse 20, un appareil de surveillance (non représenté) situé à l'extérieur du patient interroge au cours d'une étape 200, chacune des sondes 26 pour déterminer la pression dans l'anévrisme aux différents points correspondant à la position des sondes 26.

A partir de ces valeurs de pressions réelles, au cours d'une étape 210, la modélisation de l'anévrisme est mise à jour en modifiant les coefficients d'élasticité de telle sorte que la modélisation mise à jour permette de retrouver les valeurs de pression réellement obtenues. Il s'agit d'une approche de type inverse, au cours de laquelle les données fournies par les sondes sont comparées à celles issues de la modélisation numérique du même écoulement. De préférence, cette mise à jour est obtenue en faisant varier les paramètres physiques par une méthode de recherche d'extremums par la technique dite de « rétro propagation ».

Cette étape 210 fournit également des valeurs de coefficients mécaniques qui pourront être utilisées pour des patients suivants (voir étape 110).

Une fois que les coefficients ont été mis à jour, les mesures de pression de chacun des capteurs sont relevées à intervalles déterminés par le praticien. Ces relevés sont représentés par le bloc 220.

Chaque relevé de mesures permet tout d'abord de déterminer si une pression locale associée à une sonde 26 a brusquement augmenté, auquel cas il existe un risque de rupture sur la paroi artérielle 14 située le long de l'orientation du capteur, et plus généralement sur l'ensemble de la tranche à risque correspondante.

En outre, il est prévu d'utiliser le modèle numérique pour déterminer l'évolution de la pression dans l'anévrisme 12 en dehors des zones couvertes par les sondes 26 afin de détecter l'apparition de nouvelles zones fragiles en dehors des tranches à risque surveillées par les capteurs 26.

On remarquera que la prothèse de l'invention est également utilisable dans le cas d'un anévrisme résultant d'une rétractation de la paroi vasculaire. Dans ce cas, l'endoprothèse sert à écarter les parois. Les sondes 26 sont alors collées sur l'enveloppe 22, 24 en étant orientées vers l'intérieur. Les mesures de pression permettent de calculer le débit sanguin pour s'assurer que ce dernier ne faiblit pas, ce qui signifierait que l'artère est à nouveau obstruée.

## Revendications

1. Procédé de fabrication d'une endoprothèse (20) destinée à être implantée dans un anévrisme (12) d'un patient résultant d'une déformation d'une paroi vasculaire, en particulier artérielle (14), comprenant :
- une enveloppe tubulaire (22, 24) s'étendant suivant une direction (Y-Y'), et
- une pluralité de sondes (26) de pression fixées sur l'enveloppe (22, 24),
chaque sonde (26) comprenant :
- un capteur (28) pour réaliser une mesure de pression, et
- des moyens (34, 36) pour transmettre la mesure de pression à un appareil de surveillance placé à l'extérieur du patient,
les moyens (34, 36) pour transmettre la mesure dé pression de chaque sonde (26) étant adaptés pour générer un signal électromagnétique de transmission de la mesure de pression comportant au moins une caractéristique distinctive de la sonde (26) dont ces moyens (34, 36) font partie,
le procédé étant **caractérisé en ce que** le capteur de chaque sonde de la pluralité est un capteur directionnel, sa sensibilité étant maximale pour les pressions exercées perpendiculairement à sa surface de mesure et comprenant les étapes suivantes :
- obtenir une représentation visuelle de l'anévrisme (12) du patient par imagerie médicale, et
- fabriquer une enveloppe (22, 24) de forme adaptée à celle de l'anévrisme (12),
- localiser au moins un maximum local de pression (A, B) exercée par des écoulements sanguins situés dans l'anévrisme (12) sur la paroi (14) de l'anévrisme (12) du patient, et
- fixer au moins une des sondes de pression (26) sur l'enveloppe (22, 24) en orientant la sonde (26) vers l'extérieur de l'enveloppe (22, 24), de telle sorte que la sonde (26) soit orientée vers le maximum local de pression lorsque l'endoprothèse (20) est implantée dans l'anévrisme (12).

2. Procédé selon la revendication 1, **caractérisé en ce qu'**il comprend les étapes suivantes :
- déduire une modélisation de l'anévrisme (12) à partir de la représentation visuelle, et
- localiser par calcul le ou les maximum locaux de pression (A, B) exercée sur la paroi (14) de l'anévrisme (12) modélisé.

3. Procédé selon la revendication 2, **caractérisé en ce que** la représentation visuelle est une vue de scanner ou d'IRM.

4. Procédé selon la revendication 2 ou 3, **caractérisé en ce que**, pour fixer la sonde de pression (26) sur l'endoprothèse (20), le procédé comprend les étapes suivantes :
- choisir sur la modélisation de l'anévrisme (12) une configuration de pose de l'endoprothèse (20), telle que l'endoprothèse (20) s'étende le long d'une direction (X-X') d'écoulement du flux sanguin,
- déterminer une zone (27A, 27B) de fixation de capteurs (26) sur l'enveloppe (22, 24) de l'endoprothèse (20) telle que le maximum local (A, B) se trouve à la perpendiculaire de la zone de fixation (27A, 27B) par rapport à l'enveloppe (22, 24), et
- fixer la sonde de pression (26) sur la zone de fixation (27A, 27B) en orientant la sonde (26) perpendiculairement à l'enveloppe (22, 24), vers l'extérieur de l'endoprothèse (20).

5. Procédé selon la revendication 4, **caractérisé en ce qu'**il comprend les étapes suivantes :
- déterminer une tranche à risque (A, B) de l'anévrisme (12), perpendiculaire à la direction d'écoulement (X-X') et formant le maximum local,
- déterminer, le long de la direction (Y-Y') de l'enveloppe (22, 24) dans la configuration de pose de l'endoprothèse (20), un tronçon (27A, 27B) de l'enveloppe (22, 24) délimité par la tranche à risque (A, B), le tronçon formant la zone de fixation de la sonde de pression (26).

6. Procédé selon la revendication 5, **caractérisé en ce que** la tranche à risque (A, B) présente une épaisseur correspondant à une dimension que présente la sonde de pression (26) le long de la direction (Y-Y') de l'enveloppe (22, 24) lorsque la sonde (26) est fixée.

7. Procédé selon la revendication 6, **caractérisé en ce que** qu'il comprend l'étape suivante :
- fixer plusieurs sondes (26) sur le tronçon de fixation (27A, 27B) autour de la direction d'endoprothèse (Y-Y').

8. Procédé selon la revendication 7, **caractérisé en ce que** les sondes (26) sont réparties autour de la direction (Y-Y') de l'enveloppe (22, 24) à intervalles réguliers.

## Patentansprüche

1. Verfahren für die Herstellung einer Endoprothese (20), die zur Implantation in ein Aneurysma (12) eines Patienten bestimmt ist, das aus einer Verformung einer Gefäßwand, insbesondere einer arteriellen (14), resultiert, umfassend:
- eine rohrförmige Hülle (22, 24), die sich gemäß einer Richtung (Y-Y') erstreckt, und
- eine Vielzahl von Drucksonden (26), die auf der Hülle (22, 24) befestigt sind, wobei jede Sonde (26) umfasst:
- einen Sensor (28), um eine Druckmessung durchzuführen, und
- Mittel (34, 36), um die Druckmessung an ein außerhalb des Patienten hinstelltes Überwachungsgerät zu übertragen,
wobei die Mittel (34, 36) für die Übertragung der Druckmessung jeder Sonde (26) ausgebildet sind, um ein elektromagnetisches Übertragungssignal der Druckmessung zu generieren, das mindestens ein Unterscheidungsmerkmal der Sonde (26) aufweist, zu der diese Mittel (34, 36) gehören,
wobei das Verfahren **dadurch gekennzeichnet ist, dass** der Sensor jeder Sonde der Vielzahl ein Richtungssensor ist, wobei seine Empfindlichkeit für die Drücke maximal ist, die senkrecht zu seiner Messunfläche ausgeübt werden und die folgenden Schritte umfasst:
- Erhalten einer visuellen Darstellung des Aneurysmas (12) des Patienten durch medizinische Bildgebung, und
- Herstellen einer Hülle (22, 24) angepasster Form an die Form des Aneurysmas (12),
- Lokalisieren mindestens eines lokalen Druckmaximums (A, B), das von den Blutflüssen, die sich in dem Aneurysma (12) befinden, auf die Wand (14) des Aneurysmas (12) des Patienten ausgeübt wird, und
- Befestigen mindestens einer der Drucksonden (26) auf der Hülle (22, 24) bei ausrichten der Sonde (26) zum Äußeren der Hülle (22, 24) zusteuern derart, dass die Sonde (26) zum lokalen Druckmaximum ausgerichtet ist, wenn die Endoprothese (20) im Aneurysma (12) platziert ist.

2. Verfahren nach Anspruch 1, **dadurch gekennzeichnet, dass** es die folgenden Schritte umfasst:
- Ableiten einer Modellierung des Aneurysmas (12) aus der visuellen Darstellung, und
- Lokalisieren durch Berechnung des oder der auf die Wand (14) des modellierten Aneurysmas (12) ausgeübten lokalen Druckmaxima (A, B).

3. Verfahren nach Anspruch 2, **dadurch gekennzeichnet, dass** die visuelle Darstellung eine CT- oder MRT-Ansicht ist.

4. Verfahren nach Anspruch 2 oder 3, **dadurch gekennzeichnet, dass** das Verfahren zur Befestigung der Drucksonde (26) auf der Endoprothese (20) die folgenden Schritte umfasst:
- Wählen, über die Modellierung des Aneurysmas (12), einer Montagekonfiguration der Endoprothese (20) derart, dass sich die Endoprothese (20) entlang einer Strömungsrichtung (X-X') des Blutflusses erstreckt,
- Bestimmen einer Befestigungszone (27A, 27B) von Sensoren (26) auf der Hülle (22, 24) der Endoprothese (20) derart, dass sich das lokalem Maximum (A, B) an der Senkrechten der Befestigungszone (27A, 27B) im Verhältnis zur Hülle (22, 24) befindet, und
- Befestigen der Drucksonde (26) auf der Befestigungszone (27A, 27B) durch Ausrichten der Sonde (26) senkrecht zur Hülle (22, 24) zum Äußeren der Endoprothese (20).

5. Verfahren nach Anspruch 4, **dadurch gekennzeichnet, dass** es die folgenden Schritte umfasst:
- Bestimmen eines Risikoabschnitts (A, B) des Aneurysmas (12) senkrecht zur Strömungsrichtung (X-X'), der das lokale Maximum bildet,
- Bestimmen, entlang der Richtung (Y-Y') der Hülle (22, 24) in der Montagekonfiguration der Endoprothese (20), eines Teils (27A, 27B) der Hülle (22, 24), der von dem Risikoabschnitt (A, B) begrenzt ist, wobei der Teil die Befestigungszone der Drucksonde (26) bildet.

6. Verfahren nach Anspruch 5, **dadurch gekennzeichnet, dass** der Risikoabschnitt (A, B) eine Dicke aufweist, die einer Abmessung entspricht, die die Drucksonde (26) entlang der Richtung (Y-Y') der Hülle (22, 24) darstellt, wenn die Sonde (26) befestigt ist.

7. Verfahren nach Anspruch 6, **dadurch gekennzeichnet, dass** es den folgenden Schritt umfasst:
- Befestigen mehrerer Sonden (26) auf dem Befestigungsteil (27A, 27B) um die Endoprothesenrichtung (Y-Y').

8. Verfahren nach Anspruch 7, **dadurch gekennzeichnet, dass** die Sonden (26) um die Richtung (Y-Y') der Hülle (22, 24) in gleichmäßigen Intervallen verteilt sind.

## Claims

1. Method for producing an endoprosthesis (20) for implantation in an aneurysm (21) of a patient, the aneurysm resulting from a deformation of a vessel wall, in particular an arterial wall (14), comprising:
- producing a tubular envelope (22, 24) which extends according to a direction (Y-Y'), and
- fixing a plurality of pressure probes to the envelope (22, 24),
wherein each probe (26) comprises:
- a sensor (28) for carrying out a pressure measurement, and
- means (34, 36) for transmitting the pressure measurement to a monitoring device located outside the patient's body,
wherein the means (34, 36) for transmitting the pressure measurement of each probe (26) are suitable for generating an electromagnetic pressure measurement transmission signal containing at least one characteristic that is distinctive to the probe (26) of which the means (34, 36) form part,
the method being **characterized in that** the sensor of each probe of the plurality is a directional sensor, its sensitivity being maximal for pressures exerted perpendicularly to its measuring surface, and comprising the following steps:
- obtaining a visual representation of the aneurysm (12) of the patient by medical imagery,
- producing an envelope (22, 24) of a shape adapted to that of the aneurysm,
- locating at least one local pressure maximum (A, B) exerted on the wall (14) of the aneurysm (12) of the patient by blood flows within the aneurysm (12), and
- fixing at least one of the pressure probes (26) to the envelope (22, 24), the probe (26) being oriented towards the outside of the envelope so that the probe is oriented towards the local pressure maximum when the endoprosthesis (20) is implanted in the aneurysm (12).

2. Method according to claim 1, further comprising:
- deriving a model of the aneurysm (12) from the visual representation, and
- locating by calculation the local pressure maximum or maxima (A, B) exerted on the wall (14) of the modelled aneurysm (12).

3. Method according to claim 2, wherein the visual representation is a view from a scanner or MRI.

4. Method according to claim 2 or 3, wherein fixing the pressure probe (26) to the endoprosthesis (20) comprises:
- choosing a configuration for fitting of the endoprosthesis (20) from the model of the aneurysm (12), such that the endoprosthesis (20) extends along a direction (X-X') of flow of the blood flow,
- determining a zone (27A, 27B) for the fixing of sensors (26) to the envelope (22, 24) of the endoprosthesis (20), such that the local maximum (A, B) is located perpendicularly to the fixing zone (27A, 27B) relative to the envelope (22, 24), and
- fixing the pressure probe (26) to the fixing zone (27A, 27B), the probe (26) being oriented perpendicularly to the envelope (22, 24) towards the outside of the endoprosthesis (20).

5. Method according to claim 4, further comprising:
- determining an at-risk portion (A, B) of the aneurysm (12), perpendicular to the direction of flow (X-X') and forming the local maximum,
- determining along the direction (Y-Y') of the envelope (22, 24) in the configuration for fitting of the endoprosthesis (20), a section (27A, 27B) of the envelope (22, 24) delimited by the at-risk portion (A, B), the section forming the zone for fixing of the pressure probe (26).

6. Method according to claim 5, wherein the at-risk portion (A, B) has a thickness corresponding to a dimension exhibited by the pressure probe (26) along the direction (Y-Y') of the envelope (22, 24) when the probe (26) is fixed.

7. Method according to claim 6, further comprising:
- fixing a plurality of probes (26) to the fixing section (27A, 27B) around the direction (Y-Y') of the endoprosthesis.

8. Method according to claim 7, wherein the probes (26) are distributed around the direction (Y-Y') of the envelope (22, 24) at regular intervals.
